(19) Europäisches Patentamt
European Patent Office
Office européen des brevets

(11) **EP 3 518 733 B1**

(12) **EUROPÄISCHE PATENTSCHRIFT**

(45) Veröffentlichungstag und Bekanntmachung des Hinweises auf die Patenterteilung:
**01.04.2026 Patentblatt 2026/14**

(21) Anmeldenummer: **17791549.3**

(22) Anmeldetag: **24.09.2017**

(51) Internationale Patentklassifikation (IPC):
*A61B 3/13* (2006.01)   *A61F 9/007* (2006.01)
*A61B 3/103* (2006.01)   *G02B 21/00* (2006.01)
*A61B 90/20* (2016.01)   *A61B 34/20* (2016.01)
*A61B 90/00* (2016.01)

(52) Gemeinsame Patentklassifikation (CPC):
**A61B 3/13; A61B 3/1035;** A61B 90/20;
A61B 2034/2048; A61B 2090/3612; G02B 21/0012

(86) Internationale Anmeldenummer:
**PCT/DE2017/000320**

(87) Internationale Veröffentlichungsnummer:
**WO 2018/059611 (05.04.2018 Gazette 2018/14)**

(54) **VORRICHTUNG UND VERFAHREN ZUR ANZEIGE DER ASTIGMATISMUSACHSE DES AUGES**

DEVICE AND METHOD FOR DISPLAYING THE AXIS OF ASTIGMATISM OF AN EYE

DISPOSITIF ET PROCÉDÉ POUR INDIQUER L'AXE DE L'ASTIGMATISME DE L'OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2016 DE 102016011759**

(43) Veröffentlichungstag der Anmeldung:
**07.08.2019 Patentblatt 2019/32**

(73) Patentinhaber: **Chronos Vision GmbH**
**12247 Berlin (DE)**

(72) Erfinder: **SPASOVSKI, Saso**
**13088 Berlin (DE)**

(74) Vertreter: **Willems, Volker**
**Patentanwälte Weisse, Moltmann & Willems PartGmbB**
**Am Lomberg 13**
**42555 Velbert (DE)**

(56) Entgegenhaltungen:
WO-A1-2012/041349   US-A1- 2008 204 864
US-A1- 2011 019 151   US-A1- 2011 157 553
US-A1- 2011 230 751   US-A1- 2015 077 528

• BRUNA V. VENTURA ET AL: "Surgical management of astigmatism with toric intraocular lenses", ARQUIVOS BRASILEIROS DE OFTALMOLOGIA, vol. 77, no. 2, 1 April 2014 (2014-04-01), BR, pages 125 - 131, XP055435812, ISSN: 0004-2749, DOI: 10.5935/ 0004-2749.20140032

EP 3 518 733 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Bei Augenoperationen ist es wichtig, die genaue Orientierung und Position des Auges zu kennen, wobei insbesondere die genaue Kenntnis der Torsion des Auges von entscheidender Bedeutung ist. Hierzu gehören zum Beispiel die Laser-in-situ-Keratomileusis (LASIK) oder der Einsatz von torischen Intraokularlinsen bei Kataraktoperationen, die einen Astigmatismus des Auges korrigieren.

Torische Intraokularlinsen, kurz TIOL genannt, besitzen keine sphärische Geometrie. Sie werden durch eine maximale und eine minimale Krümmung (Hauptkrümmungen) entlang zweier zueinander senkrecht verlaufender Meridiane durch den Apex charakterisiert. Die Richtung mit der größten Krümmung wird durch Markierungen gekennzeichnet, die sich auf der TIOL-Oberfläche befinden und deren Ausprägung von Hersteller zu Hersteller variiert.

Die optimale torsionale Orientierung der beiden Hauptkrümmungsrichtungen der TIOL ist patientenspezifisch und hängt von der Orientierung oder Richtung der kornealen Astigmatismusachse des Auges ab. Eine vollständige Kompensation des Astigmatismus wird dann erreicht, wenn der Arzt die durch Markierungen oder Oberflächenmarkierungen auf der TIOL gekennzeichnete Richtung mit der Astigmatismusachse des Auges zur Deckung bringt.

Folglich ist es für den Erfolg der Operation von entscheidender Bedeutung, die Lage der Astigmatismusachse des Patienten während der Implantation der TIOL exakt zu kennen und dem Arzt zu präsentieren.

Für die Durchführung von Augenoperationen ist am Operationsmikroskop ein Führungs- oder Guidance-System angebracht, das dem Arzt Detaildaten des Auges zur Verfügung stellt, wie beispielsweise dessen Orientierung und Position gegenüber dem Mikroskop bzw. Operationsmikroskop.

Die intraoperative Anzeige der Astigmatismusachse des Auges während der Operation hilft dem Arzt beim Einsetzen der torischen Intraokularlinsen bzw. TIOLs, die den Astigmatismus oder astigmatischen Refraktionsfehler der Kornea des Auges kompensieren.

Es sind eine Reihe von Verfahren bekannt, die für diesen Zweck zur Anwendung kommen. Diese können unterteilt werden in manuelle Verfahren, Bildverarbeitungsverfahren und Messverfahren.

Bei den weitaus am häufigsten verbreiteten manuellen Verfahren wird die Astigmatismusachse des Patienten präoperativ während einer Diagnose mit beispielsweise einem Keratographen bestimmt und als Winkel zur Horizontalen als Messwert festgehalten. Am Operationstag wird dem Patienten in sitzender Position die Orientierung der Astigmatismusachse bzw. die Achsorientierung manuell mit einem Stempel auf der Augenoberfläche aufgebracht. Während der Operation ist diese Markierung durch das Okular des Operationsmikroskops sichtbar und kann vom Arzt als Orientierungshilfe bei der Linsenimplantation verwendet werden.

Diese Verfahren haben jedoch den Nachteil, dass die Sichtbarkeit der Markierung mit der Zeit nachlassen kann, was beispielsweise durch Spülvorgänge während der Operation verursacht werden kann. Weiterhin kann die Genauigkeit der Winkelorientierung der Markierungen durch den manuellen Charakter der Aufbringung leiden, der oftmals zu Ungenauigkeiten führt. Hinzu kommt, dass sich die Kopfausrichtung beim Anbringen der Markierung von der Kopfausrichtung während der Diagnose unterscheiden kann, sodass es hierdurch zu einem Offsetfehler kommt.

Bei den auf Bildverarbeitung basierenden Verfahren wird bei der diagnostischen Bestimmung der Astigmatismusachse gleichzeitig eine Referenzaufnahme des Auges mithilfe einer Kamera gemacht und gespeichert. Die im Bild sichtbaren skleralen Blutgefäße oder auch das Irismuster werden mit Unterstützung einer Rechnereinheit in aktuellen intraoperativen Bildern der Kamera des Operationsmikroskops automatisch gesucht, um die relative Verdrehung des Auges um die Blickrichtung zwischen Diagnose- und Operationszeitpunkt durch Matching- und Registrieralgorithmen zu bestimmen. Ist diese Verdrehung oder Torsion ermittelt, kann die aktuelle intraoperative Orientierung der Astigmatismusachse als Winkelsumme der Ausrichtung während der Diagnose und der Torsion in den Strahlengang des Mikroskops eingespielt werden und so als Orientierungshilfe für den Arzt dienen.

Dabei ergeben sich jedoch mehrere Nachteile. Zum einen kann das Fehlen von markanten Blutgefäßen und Irisstrukturen dazu führen, dass die Bildverarbeitungsalgorithmen die Torsion nur unzureichend oder gar nicht bestimmen können. In diesem Fall ist die Bestimmung der aktuellen Astigmatismusachse nicht möglich. Weiterhin können Blutungen während der Operation dazu führen, dass die während der Diagnose gut sichtbaren skleralen Blutgefäße im aktuellen Operationsbild nicht gefunden werden. Dadurch wird eine Registrierung über markante Gefäßstrukturen zwischen Diagnosebild und aktuellem Operationsbild unmöglich gemacht und die aktuelle Astigmatismusachse kann nicht ermittelt werden. Wird die Ausrichtung des Mikroskops und der damit verbundenen Mikroskopkamera intraoperativ verändert, beispielsweise um die Beobachtungsrichtung gedreht, ist eine erneute Berechnung der Torsion erforderlich, damit die korrekte Soll-Orientierung der zu implantieren TIOL angezeigt werden kann. Dieses Verfahren erfordert zwingend ein Referenzbild zum Diagnosezeitpunkt, welches nicht von allen Diagnosegeräten bereitgestellt wird.

Zur dritten Gruppe, den Messverfahren, zählen jüngere Verfahren, bei denen keine Referenzbilder aus der Diagnose erforderlich sind, da die Lage oder Orientierung der Astigmatismusachse intraoperativ erneut direkt gemessen wird, während sich der Patient bereits in liegender Position befindet. Ein derartiges Verfahren ist in der Druckschrift WO2015176699A2 offenbart, bei dem der Astigmatismus der kornealen Oberfläche mit Hilfe einer Kamera und einer Beleuchtungseinheit vermessen wird. Damit entfällt eine Registrierung per Bildverarbeitung zwischen einem Bild aus der Diagnose und einem aktuellen intraoperativen Bild der Kamera des Operationsmikroskops. Die Nachteile der zuvor beschriebenen Verfahren entfallen, da das Ergebnis nicht von der Genauigkeit von manuell aufgebrachten Markierungen oder von der gleichzeitigen Präsenz und von der Qualität von markanten Strukturen in Diagnose- und Operations-Bild abhängt.

Andere bekannte Messverfahren verwenden die Wellenfrontaberrometrie, um den Astigmatismus des Auges zu bestimmen.

US 2011/157553 A1 beschreibt ein Operationsmikroskop-System, mit einer Optik zur Erzeugung eines Bildes eines Auges während einer Augenoperation. Dabei werden mit einer Kamera Bilder des Auges aufgenommen. Ein Eye-Tracker analysiert die von der Kamera empfangenen Bilder. Mit einem Mustergenerator wird dem auf einem Display dargestellten Bild ein Muster überlagert. Das Muster wird zusammen mit dem Bild des Auges bewegt.

WO 2012/041349 A1 zeigt eine Anordnung zur Durchführung einer chirurgischen Behandlung eines Auges, die ein Diagnosegerät umfasst, das dazu eingerichtet ist, Augenstrukturdaten zu erfassen. Eine Datenverarbeitungseinheit ist dazu eingerichtet, auf der Grundlage der von dem Diagnosegerät erfassten Augenstrukturdaten ein Strukturbild zu erzeugen, das mindestens eine Abbildung einer charakteristischen Augenstruktur sowie mindestens eine relativ zu der Abbildung der charakteristischen Augenstruktur angeordnete Positionsmarkierung enthält. Eine Bilddateneinblendeeinrichtung ist dazu eingerichtet, das von der Datenverarbeitungseinheit erzeugte Strukturbild in ein von einem Operationsmikroskop während der Durchführung einer chirurgischen Behandlung des Auges erzeugtes Bild einzublenden.

US 2011/0019151 A1 beschreibt ein augenchirurgisches Mikroskopiesystem mit einer Abbildungsoptik zur Erzeugung des Bildes einer Objektebene und mit einem elektronischen Bildsensor, der das Bild der Objektebene erfasst und mit einer Recheneinheit zur Berechnung der Position des Zentrums einer Kreisstruktur eines Patientenauges verbunden ist. Die Rechnereinheit ist zur Berechnung der Position eines Patientenauges außerhalb des Zentrums der kreisförmigen Struktur ausgebildet und mit mindestens einer Markierung versehen. Die Rechnereinheit ermittelt mittels Bildverarbeitung über eine Korrelation mit einer Vergleichsinformation die Position der mindestens einen Markierung in Bezug auf das berechnete Zentrum und mittels Bildverarbeitung eine Winkelposition der mindestens einen Markierung in Bezug auf das berechnete Zentrum.

US 2011/230751 A1 offenbart eine Bildverarbeitung für die computergestützte Augenchirurgie umfassend die Erfassung eines Referenzbildes des Auges und die Anreicherung des Referenzbildes durch Einfügen zusätzlicher Kontextinformationen, die für den Chirurgen bei der Durchführung der Augenoperation hilfreich sind. Das Referenzbild wird mit einem Echtzeitbild des Auges registriert. Die Kontextinformationen werden auf der Grundlage einer Verfolgung der Augenbewegung über das Echtzeitbild des Auges gelegt, so dass die Kontextinformationen trotz einer Bewegung des Auges an der gleichen Position angezeigt werden.

US 2015/077528 A1 zeigt ein chirurgisches Führungssystem zur Unterstützung eines Chirurgen bei einem chirurgischen Eingriff an einem Patienten. Das System umfasst eine Bilderfassungsvorrichtung, die so konfiguriert ist, dass sie im Wesentlichen in Echtzeit digitale Videodaten erzeugt, die Patientenbilder von einem Auge des Patienten darstellen. Das System umfasst ferner einen Prozessor, der mit der Bilderfassungsvorrichtung gekoppelt und so konfiguriert ist, dass er die digitalen Videodaten von der Bilderfassungsvorrichtung empfängt, externe Daten von einer externen Datenquelle empfängt und zusammengesetzte digitale Bilddaten auf der Grundlage der digitalen Videodaten und der externen Daten erzeugt. Das System umfasst ferner eine mit dem Prozessor gekoppelte Anzeigevorrichtung, wobei die Anzeigevorrichtung so konfiguriert ist, dass sie dem Chirurgen aus den zusammengesetzten digitalen Bilddaten überlagerte Bilder mit Verfahrensaufforderungen entsprechend den Patientenbildern und den externen Daten anzeigt.

Die Veröffentlichung BRUNA V. VENTURA ET AL: "Surgical management of astigmatism with toric intraocularlenses", ARQUIVOS BRASILEIROS DE OFTALMOLOGIA, Bd. 77, Nr. 2, Seiten 125-131, beschreibt ein ophtalmisches Operationsmikroskop, bei dem eine Astigmatismusachse während einer Operation durch intraoperatives Eye-Tracking als überlagertes Bild in Echtzeit laufend aktualisiert wird.

Es kann jedoch vorkommen, dass der Arzt die Orientierung des Mikroskops bzw. der Mikroskopkamera verändert. Nach einer solchen Veränderung kann die Operation nicht mehr mit denselben Koordinaten hinsichtlich der Achslage des Astigmatismus fortgeführt werden oder es ergeben sich Fehler bezüglich der Orientierung der TIOL. In diesem Fall ist eine erneute Messung der Astigmatismusachse erforderlich, damit die Soll-Orientierung der zu implantierenden TIOL korrekt angezeigt werden kann.

US 2008/204864 A1 beschreibt eine Mikroskopvorrichtung mit Positionserfassung, die dazu dient, die Position des Mikroskops im Raum zu bestimmen. Die Vorrichtung umfasst ein Mikroskop und einen Beschleunigungssensor, um die dreidimensionale Position des Mikroskops zu erfassen. Die erfassten Daten werden verwendet, um die Position des Mikroskops zu bestimmen und in der Art eines Navigationssystems anzuzeigen. Aus Redundanzgründen können zusätzlich weitere Beschleunigungssensoren vorhanden sein.

In der Praxis können Änderung der Orientierung des Mikroskops während der Operation nicht ausgeschlossen werden, sodass bei den Bildverarbeitungsverfahren ein kontinuierlicher Rechenbetrieb bzw. eine kontinuierliche Messung der Astigmatismusachse bei den Messverfahren erforderlich ist. Dabei können diverse Faktoren, wie zum Beispiel Okklusionen, korneale Reflexionen, Fremdobjekte, Blutungen etc., zu einer Verfälschung der berechneten bzw. gemessenen Ergebnisse hinsichtlich der Achslage des Astigmatismus führen.

Wird die Messung der Astigmatismusachse intraoperativ und vor jeglichem Eingriff durchgeführt, wobei sich der Patient in liegender Position befindet, so kann davon ausgegangen werden, dass die gemessene Astigmatismusachse physikalisch der bei der Diagnose ermittelten Achse entspricht. Eine weitere Schwierigkeit liegt nun darin, dass einige Operationsschritte die Orientierung der Astigmatismusachse temporär oder auch dauerhaft verändern können, beispielsweise wenn mechanisch Druck auf das Auge ausgeübt wird oder Inzisionen am Limbus zur Öffnung der Augenkammer vorgenommen werden. Daraus ergibt sich, dass anschließende Messungen der Astigmatismusachse nicht mehr die ursprüngliche, d.h. präoperative Achse reproduzieren. Als Folge wird die Sollausrichtung der einzusetzenden TIOL nicht mehr korrekt angezeigt.

Aufgabe der Erfindung ist es, während der Operation die Orientierung der Astigmatismusachse in dem Bild des Auges schneller, genauer und robuster anzuzeigen, um jederzeit korrekte Informationen über die Lage bzw. Ausrichtung der Achse des zu korrigierenden Astigmatismus zur Verfügung zu stellen.

Insbesondere sollen Fehler vermieden werden, die dadurch entstehen, dass sich die Orientierung des Operationsmikroskops während der Operation verändern kann, und dass durch bestimmte Operationsschritte eine Veränderung der Astigmatismusachse erfolgen kann. Beides hat bisher im weiteren Verlauf der Operation eine fehlerhafte Information über die Orientierung der Achse des zu korrigierenden Astigmatismus zur Folge. Weiterhin soll die Robustheit bei der Ermittlung der aktuellen Achslage gesteigert werden.

Die Aufgabe wird gelöst durch die Vorrichtung gemäß Patentanspruch 1 und durch das Verfahren gemäß Patentanspruch 13. Weitere Merkmale und Details ergeben sich aus den abhängigen Ansprüchen, der Beschreibung und den Zeichnungen.

Die erfindungsgemäße Vorrichtung zur Anzeige der Astigmatismusachse des Auges umfasst eine Beobachtungseinheit zur Beobachtung des Auges, eine Anzeigeeinheit zur Anzeige der Orientierung der Astigmatismusachse des Auges in einem Bild des Auges, eine Sensoreinrichtung zur Erzeugung von Sensordaten, die eine Änderung der Ausrichtung der Beobachtungseinheit relativ zum Auge kennzeichnen, und eine Recheneinheit, wobei die Sensoreinrichtung mindestens einen Drehratensensor umfasst, der an der Beobachtungseinheit befestigt ist, um einen Drehwinkel der Beobachtungseinheit um ihre Beobachtungsrichtung zu erfassen, und die Recheneinheit dazu ausgebildet ist, mit Hilfe der Sensordaten des Drehratensensors aus der Orientierung der Astigmatismusachse in dem Bild des Auges zu einem ersten Zeitpunkt und aus dem Drehwinkel der Beobachtungseinheit um ihre Beobachtungsrichtung in dem ersten Zeitpunkt und in einem aktuellen zweiten Zeitpunkt die aktuelle Orientierung der Astigmatismusachse in dem Bild des Auges zu berechnen und der Anzeigeeinheit zur Anzeige in dem aktuellen Bild des Auges zur Verfügung zu stellen.

Hierdurch wird erreicht, dass bei einer Veränderung der Orientierung der Beobachtungseinheit gegenüber dem Auge, insbesondere bei einer Verdrehung um die Beobachtungsrichtung, die Operation mit korrekten Daten fortgeführt werden kann. Insbesondere wird nach einer Veränderung der Orientierung der Beobachtungseinheit die Orientierung der tatsächlichen Astigmatismusachse des Auges weiterhin korrekt angezeigt.

Als weiterer Vorteil ergibt sich, dass ein Diagnosebild nicht erforderlich ist. Selbst bei einer Veränderung der Orientierung der Astigmatismusachse durch Operationsschritte kann jederzeit die Orientierung der präoperativen Astigmatismusachse bzw. die Sollorientierung der zu implantierenden TIOL korrekt angegeben werden.

Darüber hinaus wird die Robustheit bei der Ermittlung der aktuellen Achslage gesteigert. Während bei direkten Messungen und bei der Bildverarbeitung Fehler und Ungenauigkeiten durch Okklusionen, Fremdobjekte, Spülvorgänge etc. auftreten können, bietet die erfindungsgemäße Lösung den Vorteil, dass sie vollständig unempfindlich gegen solche Einflüsse ist, da beispielsweise lediglich die Bewegung des Mikroskops gemessen wird.

Vorteilhafterweise umfasst die Beobachtungseinheit ein Operationsmikroskop bzw. ist als Operationsmikroskop ausgestaltet.

Insbesondere kann die Beobachtungseinheit eine Kamera zur Aufnahme von Bildern des Auges umfassen bzw. als Kamera ausgestaltet sein.

Vorteilhaft umfasst die Anzeigeeinheit ein Display bzw. ist als ein Display ausgestaltet.

Bevorzugt umfasst die Anzeigeeinheit eine Spiegelanordnung zur Einspiegelung der Achslage bzw. der Orientierung der Astigmatismusachse in den Strahlengang der Beobachtungseinheit bzw. des Operationsmikroskops. Die Anzeigeeinheit kann insbesondere als Display inklusive Spiegelanordnung zur Einspiegelung in den Strahlengang der Beobachtungseinheit bzw, des Operationsmikroskops ausgestaltet sein. Insbesondere können relativ kleine Displays, z.B. in der Größe von 2" bis 3", verwendet werden. Es können aber auch weitere Technologien zur Anzeige angewendet werden.

Vorteilhaft ist ein Speicher zur Speicherung eines Wertes bzw. Referenzwertes vorgesehen, der die Orientierung der Astigmatismusachse des Auges zu einem ersten Zeitpunkt repräsentiert. Dieser Wert charakterisiert somit die Orientierung der Astigmatismusachse, die durch die Operation korrigiert werden soll und im Bild angezeigt wird. Insbesondere wird als Referenzwert die Winkellage der Astigmatismusachse gespeichert.

Da oftmals Operationsschritte durchgeführt werden, die den Astigmatismus verändern, wird als Referenzwert, der die Referenzorientierung der Astigmatismusachse darstellt, der Winkel der gemessenen Astigmatismusachse vor der Durchführung von derartigen Operationsschritten gespeichert. Somit können astigmatismusverändernde Operationsschritte die Anzeige der Orientierung der zu korrigierenden Astigmatismusachse nicht verfälschen.

Insbesondere dient der Speicher darüber hinaus auch zur Speicherung einer Referenzorientierung der Beobachtungseinheit bzw. des Mikroskops oder der Kamera. Somit kann die Orientierung der Beobachtungseinheit bzw. des Mikroskops und/oder der Kamera gespeichert werden, die zum Zeitpunkt der Referenzmessung der Orientierung der Astigmatismusachse gültig war. Auf diese gespeicherte Orientierung der Beobachtungseinheit können sich später berechnete Mikroskop- oder Kameraorientierungen als Referenzorientierung beziehen.

Insbesondere durch die Ermittlung der Orientierung der Astigmatismusachse mit einer ersten Messung bzw. Referenzmessung, sowie die anschließende Auswertung der Sensorsignale zur Ermittlung der Orientierungsänderung des Operationsmikroskops bzw. der Beobachtungseinheit gegenüber seiner Orientierung während der Referenzmessung, ergibt sich eine erhöhte Robustheit und eine geringere Fehleranfälligkeit bei der Berechnung der aktuellen Orientierung der Astigmatismusachse im Bild des Auges als bei astigmatismusmessenden Verfahren oder der Anwendung von Bildverarbeitungsalgorithmen, deren Genauigkeit bzw. Erfolg bei der Registrierung (bzw. beim Matching) von Diagnosebild und aktuellen Kamerabildern von der Bildqualität, der Präsenz von Okklusionen und Fremdobjekten, wie zum Beispiel Operationsbesteck, Blutungen etc. negativ beeinflusst werden kann.

Gegenüber messenden Systemen ergibt sich der gleiche Vorteil, da dort die Ergebnisse ebenfalls auf der Auswertung von Kamerabildern basieren. Damit können sich alle Einflüsse auf das Bild des Auges bzw. auf dessen Sichtbarkeit negativ bemerkbar machen.

Ein weiter Vorteil besteht darin, dass die Berechnungsdauer bei der vorgeschlagenen Lösung mit Sensoren aufgrund der viel geringeren Komplexität beträchtlich kürzer ist, wodurch es zu geringeren Latenzen bei der Darstellung der aktuellen Astigmatismusachse kommt. Außerdem kann die Beleuchtung des Auges nach der Referenzmessung drastisch reduziert werden, was zu einem erhöhten Komfort für den Patienten führt.

Bevorzugt ermittelt die Sensoreinrichtung im Betrieb neben der Drehung der Beobachtungseinheit um deren Beobachtungsrichtung auch die Position der Beobachtungseinheit. Insbesondere kann mit Hilfe der Sensoreinrichtung die Drehung und die Position der Beobachtungseinheit, ermittelt werden. Die Sensoreinrichtung misst dabei den Drehwinkel des Operationsmikroskops bzw. der Beobachtungseinheit um die Beobachtungsrichtung oder um die optische Achse des Operationsmikroskops.

Bevorzugt misst die Sensoreinrichtung Winkelgeschwindigkeiten, woraus bei kontinuierlicher Überwachung und entsprechender Positionierung am Mikroskop bzw. der Beobachtungseinheit zum Beispiel dessen Verdrehung um die Beobachtungsrichtung relativ zur Referenzmessung abgeleitet werden kann.

Vorteilhafterweise umfasst die Sensoreinrichtung neben den ein oder mehreren Drehratensensoren, wie z.B. Gyroskopsensoren, ein oder mehrere inertiale Messeinheiten, auch IMU oder Inertial Measurement Unit genannt, welche unter anderem zum Beispiel auch die Gyroskopsensoren enthalten. Diese Sensoren können auch untereinander kombiniert sein. Insbesondere wird dadurch die Berechnung der relativen Lage bzw. Ausrichtung des Mikroskops bzw. der Mikroskopkamera bezogen auf eine Referenzausrichtung ermöglicht.

Bevorzugt umfasst die Sensoreinrichtung ein oder mehrere Beschleunigungssensoren, Kompasssensoren oder Richtungssensoren, die auch untereinander kombinierbar sind. Dadurch kann die Berechnung der Orientierung und Lage bzw. Position der Beobachtungseinheit, insbesondere des Mikroskops bzw. seiner Bestandteile, vereinfacht bzw. verbessert werden. Fehler, wie beispielsweise eine Drift in den Daten, werden verringert oder sogar eliminiert.

Vorteilhafterweise umfasst die Sensoreinrichtung Sensoren, die eine Verstellung der Gelenke der Beobachtungseinheit bzw. des Operationsmikroskops messen, wobei hierzu insbesondere Potentiometer oder Drehgeber verwendet werden. Dadurch ergibt sich eine besonders einfache Möglichkeit zur Lage- bzw. Richtungs- oder Positionsmessung, wobei diese Sensoren bzw. Potentiometer oder Drehgeber bevorzugt an den Gelenken des Operationsmikroskops angeordnet und besonders bevorzugt in den Gelenken integriert sind.

Insbesondere umfasst die Sensoreinrichtung ein oder mehrere Sensoren zur Ermittlung der Ausrichtung und/oder Position des Kopfes des Patienten. Insbesondere wird entweder die Ausrichtung oder die Ausrichtung und die Position des Kopfes gemessen. Insbesondere wird dabei eine Verdrehung oder Drehung des Kopfes des Patienten um die Beobachtungsrichtung oder um die optische Achse der Beobachtungseinheit bzw. des Operationsmikroskops gemessen.

Dabei kann der Patient bzw. dessen Kopf in seiner Lage getrackt oder verfolgt werden. Dadurch wird erreicht, dass eine Drehung des Kopfes des Patienten und damit seines Auges um die optische Achse der Mikroskopkamera bzw. der Beobachtungsrichtung erfasst und bei der Berechnung durch die Recheneinheit berücksichtigt wird. Das heißt, eine Drehung des Kopfes des Patienten bewirkt keine Verfälschung des Messergebnisses.

Mit Beobachtungsrichtung oder optische Achse ist insbesondere diejenige optische Achse des Operationsmikroskops oder allgemein der Beobachtungseinheit gemeint, die mit der Beobachtungsrichtung übereinstimmt, welche zum Beispiel durch die optische Achse der Eintrittsoptik des Operationsmikroskops bzw. der Beobachtungseinheit definiert ist.

Weiterhin können insbesondere Sensoren zur Messung der Ausrichtung und der Position der Operationsliege vorgesehen sein, die während der Operation den Patienten trägt und auf der der Patient und insbesondere dessen Kopf fixiert ist. Diese Sensoren können entweder einzeln oder kombiniert angeordnet sein und als Teil der gesamten Sensoreinrichtung ausgestaltet sein. Dadurch wird bei einer Änderung der Orientierung der Patientenliege während der Operation eine Verfälschung der Messergebnisse vermieden. Dies ist insbesondere sinnvoll, wenn eine relevante Verdrehung des Kopfes um die Blickrichtung oder die optische Achse der Beobachtungseinheit durch die Art der Lagerung des Patienten physikalisch unmöglich ist.

Bevorzugt wird die Ausrichtung bzw. Position der Liege des Patienten, somit auch ihre Lage oder Orientierung, mit Hilfe der Sensoren verfolgt oder getrackt.

Insbesondere ist eine Verfolgungs- bzw. Trackingeinrichtung vorgesehen, welche die Ausrichtung und Position der Beobachtungseinheit, insbesondere des Operationsmikroskops, verfolgt und die Lage bzw. Ausrichtung und Position des Kopfes des Patienten verfolgt.

Die anzuzeigende Orientierung der aktuellen Astigmatismusachse berechnet sich in diesem Fall aus der Referenzorientierung der Astigmatismusachse, der Orientierung des Mikroskops oder der Kamera bezogen auf deren Orientierung zum Referenzzeitpunkt, sowie aus der Orientierung des Kopfes oder der Patientenliege, falls der Kopf an der Liege fixiert ist, ebenfalls bezogen auf den Referenzzeitpunkt. Die Referenzlage des Kopfes bzw. der Patientenliege werden während der Referenzmessung der Astigmatismusachse analog zu Ermittlung der Referenzlage oder Referenzorientierung des Mikroskops gemessen.

Die Recheneinheit ist bevorzugt derart ausgestaltet, dass sie die Richtung bzw. Orientierung der Astigmatismusachse des Auges als Winkelsumme aus der Referenzorientierung oder Referenzwinkellage der Astigmatismusachse und der mittels der Sensoreinheit berechneten Winkellage des Operationsmikroskops bzw. der Kamera bezogen auf die optische Achse relativ zur Winkellage während der Referenzmessung bestimmt. Das heißt, es wird die Winkelsumme gebildet aus dem Winkel der Astigmatismusachse zum ersten Messzeitpunkt (=Referenzorientierung) und dem Winkel einer anschließend erfolgten Drehung von Mikroskop oder Mikroskopkamera um die Beobachtungsrichtung, die aus den Signalen der Sensoreinrichtung ermittelt wird.

Das erfindungsgemäße Verfahren zur Anzeige der Astigmatismusachse des Auges umfasst die Schritte: Bereitstellen einer Beobachtungseinheit zur Beobachtung des Auges; Bereitstellen der Orientierung der Astigmatismusachse des Auges in einem Bild der Auges zur Darstellung auf einer Anzeigeeinheit während der Beobachtung des Auges; Erfassen und Speichern von Sensordaten, die eine Änderung der Ausrichtung der Beobachtungseinheit gegenüber dem Auge kennzeichnen; wobei der Drehwinkel der Beobachtungseinheit um ihre Beobachtungsrichtung erfasst wird, und mit Hilfe der Sensordaten des Drehratensensors aus der Orientierung der Astigmatismusachse in dem Bild des Auges zu einem ersten Zeitpunkt und aus dem Drehwinkel der Beobachtungseinheit um ihre Beobachtungsrichtung in dem ersten Zeitpunkt und in einem aktuellen zweiten Zeitpunkt die aktuelle Orientierung der Astigmatismusachse in dem Bild des Auges berechnet und der Anzeigeeinheit zur Anzeige in dem aktuellen Bild des Auges zur Verfügung gestellt

Bevorzugt wird die Ausrichtung der Beobachtungseinheit gegenüber dem Auge zu dem ersten Zeitpunkt T1 und zu dem zweiten Zeitpunkt T2 ermittelt, und es wird die Orientierung der Astigmatismusachse A des Auges zum zweiten Zeitpunkt T2 aus den Sensordaten ermittelt und angezeigt.

Zur Ermittlung der anzuzeigenden Orientierung der Astigmatismusachse zum zweiten Zeitpunkt T2 wird die Orientierung der Astigmatismusachse zum ersten Zeitpunkt T1 herangezogen.

Vorteilhafterweise wird die Ausrichtung der Beobachtungseinheit verfolgt. Insbesondere erfolgt ein Tracking bzw. Verfolgen der Position und der Ausrichtung des Operationsmikroskops.

Insbesondere wird hierbei der Drehwinkel eines Operationsmikroskops um seine Beobachtungsrichtung oder um seine optische Achse ermittelt, um die Ausrichtung des Operationsmikroskops oder der Mikroskopkamera zu bestimmen.

Vorteilhaft wird die Ausrichtung und/oder Position des Kopfes des Patienten und/oder einer Patientenliege gemessen, auf der der Patient fixiert ist, um eine Verdrehung des Kopfes um die Beobachtungsrichtung bzw. die optische Achse der Beobachtungseinheit zu erfassen und bei der Berechnung der aktuellen Orientierung der Astigmatismusachse zu verwenden. Dabei kann die Orientierung des Kopfes des Patienten und/oder der Liege des Patienten auch getrackt bzw. verfolgt werden.

Insbesondere wird die aktuelle oder aktuell berechnete Orientierung der Astigmatismusachse auf einem Display dargestellt, vorteilhafterweise in einem aktuellen Bild des Auges, und/oder sie wird in den Strahlengang eines Operationsmikroskops eingespiegelt. Hierdurch kann sie bei einer Veränderung der Ausrichtung des Operationsmikroskops aktualisiert werden.

Gemäß eines nicht von der Erfindung umfassten Aspekts wird ein Verfahren zur Anzeige der Astigmatismusachse des Auges gezeigt, das folgende Schritte umfasst:

Erfassen von Bildern des Auges mit einem Operationsmikroskop und/oder einer Kamera; Erfassen eines ersten Messwertes, der die Orientierung der Astigmatismusachse im Bild des Auges zu einem ersten Zeitpunkt T1 kennzeichnet; Erfassen und Speichern der Ausrichtung des Operationsmikroskops zum ersten Zeitpunkt; Erfassen der Ausrichtung des Operationsmikroskops zu einem zweiten Zeitpunkt T2; und Anzeigen einer korrigierten Orientierung der Astigmatismusachse A des Auges im Bild des Auges zum zweiten Zeitpunkt T2, wobei die korrigierte Orientierung aus dem ersten Messwert und aus der Ausrichtung des Operationsmikroskops zum zweiten Zeitpunkt T2 bzw. aus der Ausrichtungsveränderung des Operationsmikroskops gegenüber der Ausrichtung zum Zeitpunkt T1 ermittelt wird.

Bevorzugt wird der erste Messwert erfasst, während sich der Patient in Operationslage befindet.

Vorteilhafterweise wird der erste Messwert erfasst, bevor Operationsschritte durchgeführt werden, die die Astigmatismusachse verändern.

Vorteile und Details, die im Zusammenhang mit der Vorrichtung beschrieben sind und die nachfolgend noch genauer erläutert werden, gelten auch für das erfindungsgemäße Verfahren und umgekehrt.

Nachfolgend wird die Erfindung anhand der Figuren beispielhaft beschrieben. Es zeigen:

Figur 1        eine erfindungsgemäße Vorrichtung in schematischer Darstellung;

Figur 2        eine beispielhafte Sensoranordnung mit Recheneinheit und Anzeigeeinheit in schematischer Darstellung;

Figur 3A       ein Sichtfeld eines Mikroskops, welches das Auge und die Astigmatismusachse schematisch zeigt;

Figur 3B       das Sichtfeld gemäß Figur 3A, jedoch nach einer Rotation des Operationsmikroskops um den Gravitationsvektor;

Figur 3C       das Sichtfeld gemäß Figur 3A, jedoch nach einer Rotation des Patientenkopfes oder der Operationsliege um den Gravitationsvektor.

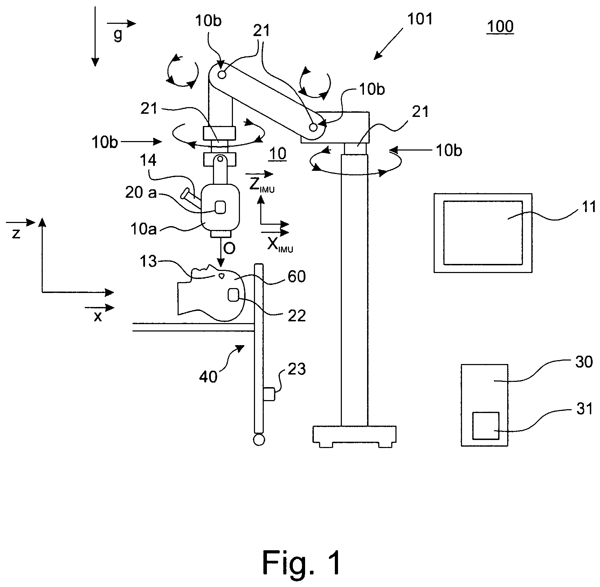

**Figur 1** zeigt als bevorzugte Ausführungsform der Erfindung eine Vorrichtung 100, die zur Anzeige des Auges mit seiner Astigmatismusachse bei Augenoperationen dient. Dabei dient ein Mikroskop bzw. Operationsmikroskop 10 als Beobachtungseinheit zur Beobachtung des Auges 13 eines Patienten.

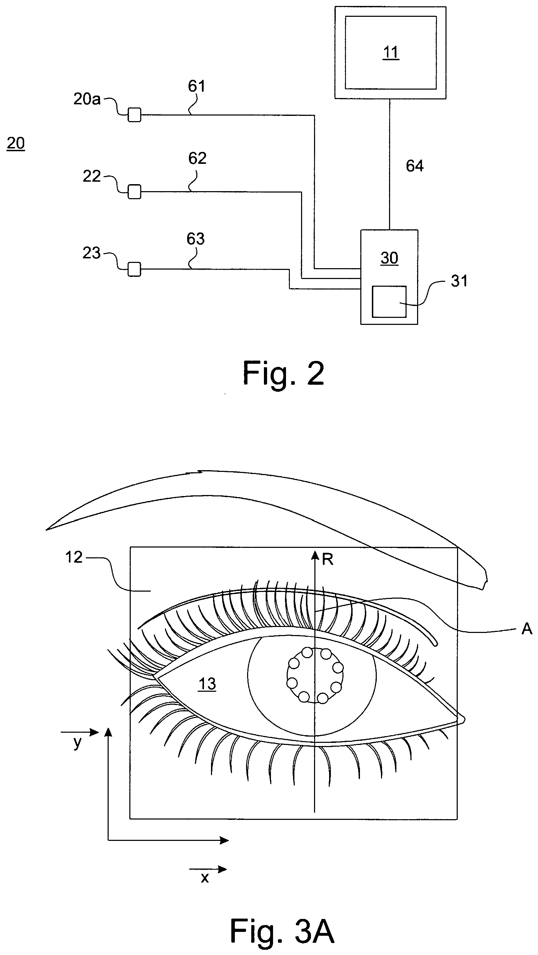

Eine Kamera ist als integrierte Komponente des Mikroskopkopfes 10a ausgestaltet und nimmt Bilder 12 des Auges 13 auf (siehe Figur 3A). Eine Anzeigeeinheit 11 dient zur Anzeige der Orientierung R der Astigmatismusachse A des Auges 13 in den dargestellten bzw. aufgenommenen Bildern 12 des Auges, das durch das Operationsmikroskop 10 erfasst wird

Ein Sensor 20a ermöglicht die Bestimmung der Ausrichtung bzw. der Lage des Operationsmikroskops 10. Der Sensor 20a kann als inertiale Messeinheit bzw. IMU ausgestaltet sein und misst kontinuierlich Beschleunigungen und Winkelgeschwindigkeiten in je drei Freiheitsgraden. Er kann aber erfindungsgemäß auch als separater Drehratensensor bzw. Gyroskopsensor ausgestaltet sein.

Weiterhin ist eine Recheneinheit 30 vorgesehen, die die aktuelle Orientierung R' der Astigmatismusachse A in dem jeweiligen aktuellen Bild 12 des Auges 13 berechnet (siehe Figuren 3A bis 3C). Die Berechnung erfolgt erfindungsgemäß aus der Orientierung R der Astigmatismusachse A des Auges 13 zu einem ersten Zeitpunkt T1, und der aus den Sensordaten ableitbaren jeweiligen Ausrichtung des Operationsmikroskops 10 in dem ersten Zeitpunkt T1 und in einem zweiten Zeitpunkt T2, welcher der aktuelle Zeitpunkt ist. Das heißt, aus der ursprünglich korrekt bestimmten Orientierung der Astigmatismusachse im Bild 12 des Auges 13 und der anschließenden Veränderung der Ausrichtung des Operationsmikroskops 10 wird die Orientierung R' der Astigmatismusachse im aktuellen Bild des Auges bestimmt.

Die Recheneinheit 30 kann aus mehreren Komponenten bestehen. Beispielsweise handelt es sich um ein oder mehrere Mikrocontroller in Kombination mit einem PC. Die Mikrocontroller lesen dabei die Sensoren aus und leiten die Daten entweder direkt oder verarbeitet an den PC, der die finalen Berechnungen durchführt und die Ergebnisse an die Anzeigeeinheit weiterleitet.

Die Recheneinheit 30 ist an die Anzeigeeinheit 11 gekoppelt um der Anzeigeeinheit 11 die aktuelle Orientierung R' der Astigmatismusachse zur Verfügung zu stellen.

Das Operationsmikroskop 10 umfasst eine Halterung 101 in Form eines Tragegestells mit mehreren relativ zueinander bewegbaren Elementen, die durch Gelenke 10b bewegbar miteinander verbunden sind. Den zentralen Teil des Operationsmikroskops 10 bildet ein Mikroskopkopf 10a mit der integrierten Kamera, der bewegbar an der Halterung 101 befestigt ist.

Am Operationsmikroskop 10 befindet sich ein Okular 14, durch das der Arzt bei der Operation das durch das Mikroskop vergrößerte Bild des Auges 13 des Patienten betrachtet.

Vor und während der Operation wird das bewegbare Operationsmikroskop 10 mit seiner Kamera auf das Auge 13 des Patienten ausgerichtet. Diese Richtung bildet die Beobachtungsrichtung O und entspricht der optischen Achse der Lichteintrittsoptik des Operationsmikroskops 10 sowie der im Mikroskopkopf 10a integrierten Kamera.

Mit dem am Mikroskopkopf 10a befestigten Sensor 20a werden Veränderungen der Lage und/oder Ausrichtung der Kamera gemessen. Dabei wird eine Drehung des Operationsmikroskops 10 um die Beobachtungsrichtung O erfasst. Sofern das Operationsmikroskop 10 senkrecht von oben auf das Auge 13 des liegenden Patienten ausgerichtet ist, entspricht die Beobachtungsrichtung O der Richtung des Gravitationsrektors g, die in Figur 1 entgegengesetzt zur Z-Richtung verläuft.

Der als inertiale Messeinheit oder erfindungsgemäß als Drehraten- bzw. Gyroskopsensor ausgestaltete Sensor 20a misst die Drehung bzw. Winkelgeschwindigkeit oder erfindungsgemäß die Drehrate der Kamera und des Mikroskopkopfes 10a um die Beobachtungsrichtung O, hier also um die Z-Richtung. Mit Hilfe der Recheneinheit 30 lässt sich daraus beispielsweise die relative Winkelstellung der Kamera um die Beobachtungsrichtung ermitteln.

Zusätzlich zum hier gezeigten Sensor 20a sind in dem bevorzugten Ausführungsbeispiel Sensoren 21 angeordnet, die die Stellung der Gelenke 10b des Operationsmikroskops 10 messen. Die Sensoren 21 sind als Potentiometer oder Drehgeber ausgestaltet, die in den Gelenken 10b des Operationsmikroskops 10 bzw. dessen Halterung 101 angeordnet sind. Dadurch werden Drehbewegungen der Teileelemente der Halterung 101 relativ zueinander erfasst, aus denen sich die Lage und Ausrichtung des Operationsmikroskops 10 bzw. seiner Kamera 10a bestimmen lässt.

Die Sensoren 21 zur Messung der Stellung der Gelenke 10b können zusätzlich oder alternativ zu den ein oder mehreren Drehraten- oder Gyroskopsensoren 20a angeordnet sein.

Die hier dargestellte bevorzugte Ausführungsform der Erfindung umfasst zusätzlich ein oder mehrere Sensoren 22, die am Kopf 60 des Patienten befestigt sind und eine Drehung des Kopfes 60 des Patienten um die Beobachtungsrichtung O messen bzw. erfassen. Die Sensoren 22 sind bevorzugt Drehraten- oder Gyroskopsensoren, oder sie sind als inertiale Messeinheiten bzw. IMU ausgestaltet. Dadurch kann eine Drehung des Kopfes 60 des Patienten während der Operation bei der Bestimmung der Orientierung der Astigmatismusachse A des Auges in dem angezeigten Bild 12 des Auges berücksichtigt werden.

Eine Operationsliege 40 dient dazu, den Patienten während der Operation zu tragen. Der Patient ist dabei auf der Operationsliege 40 üblicherweise so befestigt, dass sich der Kopf 60 des Patienten nicht bewegen kann bzw. an der Operationsliege 40 fixiert ist.

Die hier als bevorzugtes Beispiel gezeigte Vorrichtung umfasst weiterhin einen Sensor 23, der die Ausrichtung und oder die Position der Operationsliege 40 misst. Von besonderer Bedeutung sind dabei Drehungen der Liege um die Gravitationsrichtung Z bzw. um die Beobachtungsrichtung O. D.h., die Operationsliege 40 kann hinsichtlich ihrer Position und Lage oder Orientierung mit Hilfe der ein oder mehreren Sensoren 23 verfolgt werden, falls eine Orientierungsänderung der Operationsliege 40 während der Operation nicht ausgeschlossen werden kann. Dies ist insbesondere dann sinnvoll, wenn eine relevante Verdrehung des Kopfes 60 um die optische Achse der beobachtenden Mikroskopkamera durch die Art der Lagerung des Patienten physikalisch unmöglich ist, das heißt, wenn der Kopf 60 des Patienten so an der Operationsliege 40 befestigt ist, dass er sich relativ zu dieser nicht bewegen kann.

Somit kann der Kopf 60 des Patienten, analog zum Operationsmikroskop 10 bzw. der im Mikroskopkopf 10a integrierten Kamera, ebenfalls räumlich verfolgt bzw. getrackt werden. Ist eine Verdrehung des Kopfes des Patienten relativ zur Liege durch die Art der Lagerung unmöglich, dann ist es ausreichend, wenn entweder der Kopf oder die Liege mit einem Sensor ausgestattet ist.

Die Sensoren 20a, 21, 22, 23 können einzeln oder miteinander kombiniert angeordnet sein, um die relative Drehung zwischen der Operationskamera und dem Auge 13 um die Beobachtungsrichtung O zu erfassen. Sie bilden einzeln oder in Kombination eine Sensoreinrichtung 20. Diese ist so an die Recheneinheit 30 gekoppelt, dass die Signale der Sensoren 20a, 21, 22, 23 an die Recheneinheit 30 übertragen werden. Dies erfolgt mittels einer elektrischen Verbindung, die auch drahtlos sein kann.

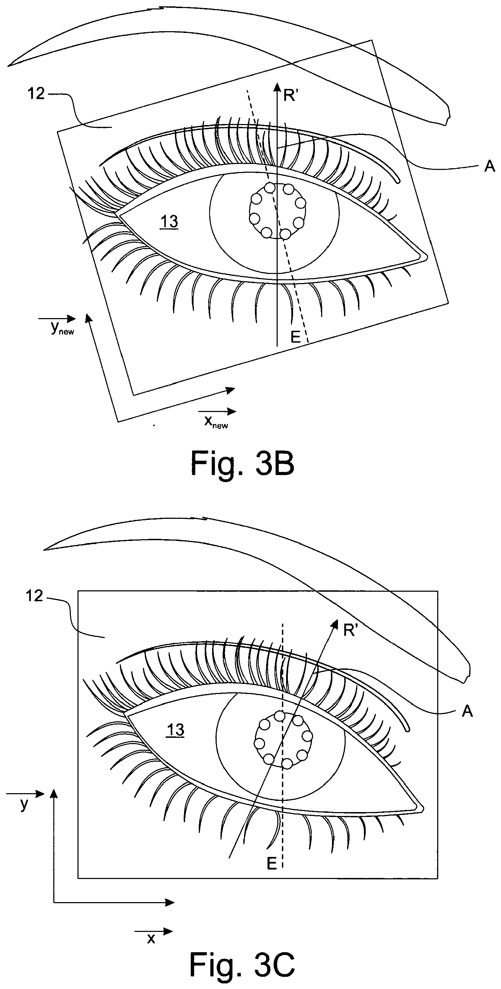

Die Recheneinheit 30 berechnet die aktuelle Orientierung R' der Astigmatismusachse A im Bild 12 des Auges 13 im Verlauf der Augenoperation. Solange keine Veränderung der Ausrichtung oder der Lage des Operationsmikroskop 10 in Bezug auf das Auge 13 des Patienten erfolgt, entspricht die aktuelle Orientierung R' der Astigmatismusachse A der tatsächlichen Orientierung R der Astigmatismusachse. Dies setzt jedoch voraus, dass sich die tatsächliche Orientierung der Astigmatismusachse, die vor der Operation diagnostisch ermittelt wurde, nicht durch bestimmte Operationsschritte verändert hat. Die Berechnung wird weiter unten unter Bezugnahme auf die Figuren 3A, 3B, 3C noch genauer erläutert.

Die Recheneinheit 30 beinhaltet einen Speicher 31, der unter anderem die Orientierung R der Astigmatismusachse A des Auges als Referenzwert für die späteren Berechnungen speichert. Ebenso sind in dem Speicher die durch die Sensoreinrichtung 20 ermittelten Messwerte abgelegt, die zur Berechnung der aktuellen Orientierung R' dienen.

Die Anzeigeeinheit 11 ist an die Recheneinheit 30 gekoppelt, um die berechnete Orientierung R' der Astigmatismusachse A in dem Bild 12 des Auges 13 anzuzeigen. Die Anzeigeeinheit 11 ist zum Beispiel ein Display, dass das Bild wiedergibt, welches von der im Mikroskopkopf 10a integrierten Kamera aufgenommen wird. Zusätzlich zu dem so aufgenommenen Bild wird die Astigmatismusachse A in der von der Recheneinheit 30 berechneten aktuellen Orientierung R' dargestellt bzw. dem Bild 12 des Auges 13 überlagert.

Es ist aber auch möglich, dass die Anzeigeeinheit 11 durch das Okular des Operationsmikroskops 10 betrachtet werden kann oder in das Operationsmikroskop 10 integriert ist.

**Figur 2** zeigt im Detail die Recheneinheit 30, die Anzeigeeinheit 11, sowie die Sensoren 20a, 22 und 23, wie sie oben unter Bezugnahme auf Figur 1 beschrieben sind. Die Sensoren 20a, 22, 23 bilden die Sensoreinrichtung 20 bzw. sind Teil davon, und sind als Drehraten- bzw. Gyroskopsensoren oder als IMUs ausgestaltet. Der mindestens eine Sensor 20a

ist am Mikroskopkopf 10a angeordnet, um dessen Ausrichtung oder Lage mit Hilfe der Recheneinheit zu ermitteln, während die ein oder mehreren Sensoren 22 bzw. 23 am Kopf 30 des Patienten bzw. an der Operationsliege 40 befestigt sind, um die Ermittlung der Ausrichtung oder Lage des Kopfes 60 bzw. der Operationsliege 40 zu ermöglichen.

Die Recheneinheit 30 liest kontinuierlich die von den Gyroskopen oder IMUs gemessenen Winkelgeschwindigkeiten aus und errechnet daraus die Ausrichtung bezogen auf eine Referenzausrichtung.

Die Sensoren der Sensoreinrichtung 20 sind über elektrische Verbindungen 61, 62, 63 mit der Recheneinheit 30 verbunden. Die elektrischen Verbindungen können auch drahtlos ausgestaltet sein. Sie dienen zur Übertragung der Sensorsignale an die Recheneinheit 30.

Die Recheneinheit 30 ist durch eine weitere elektrische Verbindung 64, die zum Beispiel drahtlos ausgestaltet ist, mit der Anzeigeeinheit 11 verbunden. Die Recheneinheit 30 erzeugt Signale zum Ansteuern der Anzeigeeinheit 11, um dort die jeweils aktuelle Orientierung R' der Astigmatismusachse A im Bild 12 des Auges 13 anzuzeigen, die aus den von den Sensoren gemessenen Werten berechnet wird.

**Figur 3A** zeigt das Sichtfeld der Mikroskopkamera, in dem sich das Bild 12 des Auges 13 des Patienten befindet. Das Sichtfeld wird auf der Anzeigeeinheit 11 dargestellt (siehe Figur 1). Ebenfalls im Bild 12 dargestellt ist die Astigmatismusachse A des Auges 13, die in dem dargestellten Beispiel annähernd in Y-Richtung ausgerichtet ist.

**Figur 3B** zeigt das Sichtfeld der Mikroskopkamera, wobei hier jedoch eine Verdrehung des angezeigten Bildes 12 um die Z-Richtung erfolgt ist, die senkrecht zur Bildebene verläuft. Diese Verdrehung des Bildes um die Z-Richtung wird hier zum Beispiel durch eine Verdrehung des Operationsmikroskops 10 um die Beobachtungsrichtung O entgegen dem Uhrzeigersinn verursacht (siehe Fig. 1). Durch die Verdrehung des Bildes 12 ist das Auge 13 im Bild 12 im Uhrzeigersinn verdreht dargestellt.

Ohne die erfindungsgemäße Berechnung und Korrektur der angezeigten Orientierung der Astigmatismusachse A würde sich diese mit dem Bild nicht mitdrehen und somit fehlerhaft angezeigt werden. Diese fehlerhaft angezeigte Orientierung ist durch die gestrichelte Linie E in Figur 3D dargestellt. Der Fehler, der sich daraus ergeben würde, entspricht dem Drehwinkel $\alpha$, d.h. dem Winkel der Drehung des Operationsmikroskops 10 bzw. des Bildes 12.

$$\alpha = \text{Winkel}\,(\vec{y}, \vec{y}_{new}) \quad \text{bzw.} \quad \alpha = \text{Winkel}\,(\vec{x}, \vec{x}_{new})$$

Die Recheneinheit 30 berechnet nun aus den von der Sensoreinrichtung 20 gelieferten Signalen die neue bzw. korrigierte Orientierung R' oder Winkellage der Astigmatismusachse A im Bild 12, sodass diese in Bezug auf das Auge 13, d.h. relativ zum Auge 13, korrekt dargestellt wird. Der Korrekturwinkel entspricht dem Winkel $\alpha$, d.h. dem Winkel, um den sich das Bild 12 gegenüber seiner Ausgangslage gedreht hat.

**Figur 3C** zeigt beispielhaft den Fall, dass sich der Kopf 60 des Patienten um die Beobachtungsrichtung O gedreht hat. Dies kann durch den Patienten selbst und/oder durch eine Drehung der Operationsliege 40 um die Beobachtungsrichtung O verursacht worden sein. In diesem Beispiel wird angenommen, dass keine Veränderung der Ausrichtung des Operationsmikroskops 10 erfolgt ist.

Als Folge der Drehung des Kopfes 60 entspricht die Ausrichtung des Auges 13 im Bild 12 bzw. der Astigmatismusachse nicht mehr der ursprünglichen Ausrichtung, d.h. das Auge 13 ist im Bild 12 verdreht dargestellt. Ohne die erfindungsgemäße Berechnung der Orientierung der Astigmatismusachse A würde diese wiederum fehlerhaft dargestellt, wie durch die gestrichelte Linie E gezeigt. D.h., sie würde in diesem Beispiel weiterhin annähernd parallel zur Richtung der y-Achse des Bildes verlaufend angezeigt werden, so wie ihre Lage im Bild 12 ursprünglich bzw. vor der Verdrehung des Kopfes 60 ermittelt wurde (siehe Figur 3A).

Mithilfe der Sensoreinrichtung 20 und der Recheneinheit 30 erfolgt nun eine Korrektur dieses Fehlers. Die tatsächliche, aktuelle Ausrichtung bzw. Orientierung der Astigmatismusachse A des Auges wird berechnet, indem ihre Winkellage um den Winkel $\beta$ korrigiert wird.

Dieser Winkel $\beta$ entspricht dem Drehwinkel des Kopfes 60 um die Beobachtungsrichtung O.

$$\beta = \text{Winkel}_{Rot}\,(\text{Kopf})$$

Ist der Kopf derart gelagert, dass eine Verdrehung relativ zur Patientenliege aufgrund einer Fixierung nicht möglich ist, so kann auf einen Kopfsensor verzichtet werden. Stattdessen kann zur Feststellung einer Drehung des Kopfes und damit des Auges ein an der Liege angebrachter Sensor zur Anwendung kommen. In einem solchen Fall entspricht $\beta$ dem Drehwinkel der Patientenliege.

$$\beta = \text{Winkel}_{Rot}\,(\text{Liege})$$

Ein zusätzlicher Kopfsensor wäre redundant, da dieser die gleiche Drehung bzw. die gleiche Drehrate oder Winkelgeschwindigkeit um die Beobachtungsrichtung wie der Sensor an der Liege messen würde, d.h. es ergibt sich der gleiche Winkel $\beta$

$$\beta = \text{Winkel}_{\text{Rot}}\,(\text{Liege}) = \text{Winkel}_{\text{Rot}}\,(\text{Kopf})$$

Als Folge dieser Berechnung wird die Astigmatismusachse A im Bild 12 in der Richtung R' angezeigt. Die Orientierung R' entspricht aufgrund der erfolgten Berechnung bzw. der tatsächlichen Orientierung der Astigmatismusachse A des Auges, die zuvor diagnostiziert wurde.

Nachfolgend werden die einzelnen Schritte des Verfahrens erläutert:

Um bei einer Augenoperation die Astigmatismusachse des Auges anzuzeigen, wird zunächst ein Bild 12 des Auges 13 mit dem Operationsmikroskop 10 erfasst. Dies erfolgt zu einem Zeitpunkt T1, in dem noch keine Drehung des Operationsmikroskops 10 relativ zum Auge 13 des Patienten stattgefunden hat. In diesem Zustand wird ein erster Messwert erfasst, der die Orientierung R der Astigmatismusachse A in den Bild 12 kennzeichnet. Somit entspricht die Orientierung R der Astigmatismusachse A zum Zeitpunkt T1 im Bild 12 des Auges 13 der tatsächlichen Orientierung (**siehe Figur 3A**). In dem in Figur 3A dargestellten Beispiel sind das ca. 90 Grad gegenüber der x-Achse des Bildes

Nun wird die Winkelposition OP1 des Mikroskopkopfes 10a um die Beobachtungsrichtung zu diesem ersten Zeitpunkt mit beispielsweise 0 Grad initialisiert und als Referenzorientierung gespeichert,

d.h. OP1 := 0 Grad.

Während der Operation wird nun die Winkelposition bzw. Drehung OP2 des Mikroskopkopfes 10a und damit der integrierten Kamera um die Beobachtungsrichtung erneut ermittelt, d.h. zu einem späteren, zweiten Zeitpunkt T2. Zu diesem Zeitpunkt wurde der Mikroskopkopf 10a und damit die integrierte Kamera um zum Beispiel 30 Grad gegen den Uhrzeigersinn um die z-Achse gedreht. Die neue Ausrichtung der Kamera bezogen auf die Ausrichtung zum Zeitpunkt T1 entspricht dem Winkel OP2-OP1, der in diesem Fall plus 30 Grad beträgt,

$$\text{d.h. OP2 - OP1 = +30 Grad.}$$

Das Vorzeichen ist positiv, da die Drehung des Operationsmikroskops 10 um die z-Achse gegen den Uhrzeigersinn erfolgte. Der Winkel der Drehung des Operationsmikroskops 10 beträgt:

$$\alpha = \Delta OP = OP2 - OP1,$$

das heißt in diesem Beispiel: $\Delta OP = +30$ Grad - 0 Grad, also

$$\alpha = +30 \text{ Grad}$$

Dadurch hat sich die Orientierung des Auges 13 im Bild um 30 Grad im Uhrzeigersinn gedreht (siehe Figur 3B). Das heißt die erfolgte Drehung des Auges im Bild 12 zum Zeitpunkt T2 beträgt:

$$\Delta A = -\Delta OP, \text{ hier also } \Delta A = -30 \text{ Grad}$$

Anschließend erfolgt die Berechnung der korrigierten Orientierung R' der Astigmatismusachse A in dem Bild 12. Hierzu wird zunächst der erste Messwert herangezogen, der die Orientierung R der Astigmatismusachse A im Bild 12 zum ersten Zeitpunkt T1 kennzeichnet. In diesem Beispiel also R = 90 Grad. Zum Winkel R, der die ursprüngliche Orientierung R der Astigmatismusachse zum Zeitpunkt T1 kennzeichnet, wird der Winkel $\Delta A$ addiert:

$$R' = R + \Delta A, \text{ bzw.}$$

$$R' = R - \Delta OP = R - (OP2 - OP1) = R - \alpha = 90 \text{ Grad} - 30 \text{ Grad} = 60 \text{ Grad}$$

Die so berechnete Orientierung R' der Astigmatismusachse A des Auges 13 zum Zeitpunkt T2 wird mittels der Anzeigeeinrichtung im Bild 12 des Auges angezeigt.

Der Winkel der erfolgten relativen Drehung zwischen dem Operationsmikroskop 10 oder der Kamera in 10a zum Auge 13 kann sich aber, wie oben in Figur 3C dargestellt, nicht nur aus einer Drehung des Operationsmikroskops 10 mit der Kamera ergeben, sondern auch aus einer Drehung des Kopfes 60 des Patienten.

In diesem Fall ergibt sich R' wie folgt:

$$R´ = R + \beta$$

wobei β den Winkel der erfolgten Drehung des Kopfes 60 um die z-Richtung bzw. um die Richtung des Gravitationsvektors g kennzeichnet.

Unter Berücksichtigung sämtlicher Drehungen ergibt sich der korrigierte Winkel für die Darstellung der Astgmatismusachse R' im Bild 12 des Auges 13 aus:

$$R´ = R - \alpha + \beta$$

Die Erfassung der Winkellage und Berechnung von R' kann auch mehrmals oder kontinuierlich erfolgen, d.h. dass die Position bzw. Ausrichtung des Operationsmikroskops 10 im Verlauf der Operation verfolgt bzw. getrackt wird. Das heißt, die Ausrichtung bzw. Lage und Position des Operationsmikroskops 10, des Kopfes 60 des Patienten sowie der Operationsliege 40 werden vorteilhafterweise während der Operation mehrmals gemessen und auf diese Weise verfolgt, so das zu jedem Zeitpunkt der Operation die Orientierung R' der Astigmatismusachse A im Bild 12 des Auges korrekt dargestellt wird, unabhängig davon, welche Verdrehungen um die Beobachtungsrichtung O zwischenzeitlich stattgefunden haben.

Sofern sich durch entsprechende Operationseingriffe die Lage bzw. Ausrichtung der Astigmatismusachse des Auges verändert, wie es beispielsweise bei Inzisionen am Irisrand der Fall sein kann, wird dennoch die zu zuvor bestimmte, präoperative Lage der Astigmatismusachse A auf der Anzeigeeinheit 11 dargestellt, da während der Operation keine erneute Messung der Astigmatismusachse am Auge erfolgt. Stattdessen wird stets von der ursprünglichen Astigmatismusachse A mit der ursprünglichen Orientierung R im dargestellten Bild ausgegangen, sowie von der anschließend während der Operation stattfindenden relativen Drehung zwischen Operationsmikroskop 10 bzw. der Kamera in 10a und dem Auge 13 um die Beobachtungsrichtung Z.

Die erfindungsgemäße Vorrichtung und das erfindungsgemäße Verfahren bieten den Vorteil, dass ein Diagnosebild nicht erforderlich ist. Trotz einer möglichen Veränderung der Orientierung der Astigmatismusachse durch Operationsschritte kann jederzeit die Orientierung der präoperativen Achse bzw. die Soll-Orientierung der zu implantierenden IOL angezeigt werden. Die Ermittlung der Orientierung der Astigmatismusachse A durch eine Referenzmessung sowie anschließende Auswertung von Sensorsignalen ist weit weniger komplex und fehleranfällig als eine kontinuierliche, kamerabasierte direkte Messung oder die Anwendung von Bildverarbeitungsalgorithmen, deren Genauigkeit bei der Registrierung von Diagnosebild mit aktuellem Kamerabildern von der Bildqualität, der Präsenz von Fremdobjekten, wie zum Beispiel Operationsinstrumenten, Okklusionen oder Blutungen usw., negativ beeinflusst werden kann.

**Patentansprüche**

1.  Vorrichtung eingerichtet zur Anzeige der Astigmatismusachse des Auges, umfassend

    eine Beobachtungseinheit (10) zur Beobachtung des Auges (13);
    eine Anzeigeeinheit (11) eingerichtet zur Anzeige der Orientierung (R) der Astigmatismusachse (A) des Auges (13) in einem Bild (12) des Auges (13),
    eine Sensoreinrichtung (20) eingerichtet zur Erzeugung von Sensordaten, die eine Änderung der Ausrichtung der Beobachtungseinheit (10) relativ zum Auge (13) kennzeichnen, und
    eine Recheneinheit (30),
    **dadurch gekennzeichnet, dass**
    die Sensoreinrichtung (20) mindestens einen Drehratensensor (20a) umfasst, der an der Beobachtungseinheit (10) befestigt ist, und dazu eingerichtet ist, einen Drehwinkel der Beobachtungseinheit (10) um ihre Beobachtungsrichtung (O) zu erfassen, und
    die Recheneinheit (30) dazu ausgebildet ist, mit Hilfe der Sensordaten des Drehratensensors (20a) aus der Orientierung (R) der Astigmatismusachse (A) in dem Bild (12) des Auges (13) zu einem ersten Zeitpunkt (T1) und aus dem Drehwinkel der Beobachtungseinheit (10) um ihre Beobachtungsrichtung (O) in dem ersten Zeitpunkt (T1) und in einem aktuellen zweiten Zeitpunkt (T2) die aktuelle Orientierung (R') der Astigmatismusachse (A) in dem Bild (12) des Auges (13) zu berechnen und der Anzeigeeinheit (30) zur Anzeige in dem aktuellen Bild (12) des Auges (13) zur Verfügung zu stellen.

2.   Vorrichtung gemäß Anspruch 1, **dadurch gekennzeichnet, dass** die Beobachtungseinheit (10) ein Operationsmikroskop umfasst oder als solches ausgestaltet ist.

3.   Vorrichtung nach Anspruch 1 oder 2, **dadurch gekennzeichnet, dass** die Beobachtungseinheit (10) eine Einrichtung (10a) zur Aufnahme von Bildern des Auges (13) umfasst oder als solche ausgestaltet ist.

4.   Vorrichtung gemäß einem der vorhergehenden Ansprüche, **dadurch gekennzeichnet, dass** die Anzeigeeinheit (11) als Display ausgestaltet ist.

5.   Vorrichtung gemäß einem der vorhergehenden Ansprüche, **dadurch gekennzeichnet, dass** die Anzeigeeinheit (11) eine Spiegelanordnung zur Einspiegelung in den Strahlengang der Beobachtungseinheit (10) umfasst.

6.   Vorrichtung gemäß einem der vorhergehenden Ansprüche,
**gekennzeichnet durch** einen Speicher (31) zur Speicherung eines Referenzwertes, der die Orientierung (R) der Astigmatismusachse A des Auges (13) zu dem ersten Zeitpunkt (T1) repräsentiert, und / oder zur Speicherung einer Referenzorientierung der Beobachtungseinheit (10).

7.   Vorrichtung nach einem der vorhergehenden Ansprüche, **dadurch gekennzeichnet, dass** die Sensoreinrichtung (20) die Position der Beobachtungseinheit (10) ermittelt.

8.   Vorrichtung gemäß einem der vorhergehenden Ansprüche, **dadurch gekennzeichnet, dass** die Sensoreinrichtung (20) ein oder mehrere Inertiale Messeinheiten umfasst.

9.   Vorrichtung gemäß einem der vorhergehenden Ansprüche, **dadurch gekennzeichnet, dass** die Sensoreinrichtung (20) ein oder mehrere Sensoren aus der Gruppe Beschleunigungssensoren, Richtungssensoren, Kompasssensoren und Sensoren (21) zur Messung der Stellung der Gelenke (10b) der Beobachtungseinrichtung (10) umfasst.

10.   Vorrichtung gemäß einem der vorhergehenden Ansprüche, **dadurch gekennzeichnet, dass** die Sensoreinrichtung (20) ein oder mehrere Sensoren (22) zur Ermittlung der Ausrichtung und/oder Position des Kopfes (60) des Patienten umfasst.

11.   Vorrichtung gemäß einem der vorhergehenden Ansprüche, **dadurch gekennzeichnet, dass** die Sensoreinrichtung ein oder mehrere Sensoren (23) zur Ermittlung der Ausrichtung und/oder Position einer Operationsliege (40) umfasst.

12.   Vorrichtung gemäß einem der vorhergehenden Ansprüche,
**gekennzeichnet durch** eine Einrichtung zur Verfolgung der Position und/oder der Ausrichtung der Beobachtungseinheit (10) und/oder der Ausrichtung und/oder der Position des Kopfes (60) des Patienten.

13.   Verfahren zur Anzeige der Astigmatismusachse des Auges,
**gekennzeichnet durch** die Schritte:

Bereitstellen einer Beobachtungseinheit (10) zur Beobachtung des Auges (13);
Bereitstellen der Orientierung R der Astigmatismusachse des Auges (13) in einem Bild (12) der Auges (13) zur Darstellung auf einer Anzeigeeinheit (11) während der Beobachtung des Auges (13);
Erfassen und Speichern von Sensordaten, die eine Änderung der Ausrichtung der Beobachtungseinheit (10) gegenüber dem Auge (13) kennzeichnen;
**dadurch gekennzeichnet, dass**
der Drehwinkel der Beobachtungseinheit (10) um ihre Beobachtungsrichtung (O) durch einen Drehratensensor (20a) erfasst wird, und
mit Hilfe der Sensordaten des Drehratensensors (20a) aus der Orientierung (R) der Astigmatismusachse (A) in dem Bild (12) des Auges (13) zu einem ersten Zeitpunkt (T1) und aus dem Drehwinkel der Beobachtungseinheit (10) um ihre Beobachtungsrichtung (O) in dem ersten Zeitpunkt (T1) und in einem aktuellen zweiten Zeitpunkt (T2) die aktuelle Orientierung (R') der Astigmatismusachse (A) in dem Bild (12) des Auges (13) berechnet und der Anzeigeeinheit (30) zur Anzeige in dem aktuellen Bild (12) des Auges (13) zur Verfügung gestellt wird.

14.   Verfahren nach Anspruch 13, **dadurch gekennzeichnet, dass** die Ausrichtung der Beobachtungseinheit (10) verfolgt wird.

**15.** Verfahren nach Anspruch 13 oder 14, **dadurch gekennzeichnet, dass** die Ausrichtung und/oder Position des Kopfes (60) eines Patienten und/oder einer Patientenliege (40) gemessen wird, um eine Verdrehung des Kopfes (60) um die Beobachtungsrichtung (O) oder die optische Achse der Beobachtungseinheit (10) zu erfassen und bei der Berechnung der aktuellen Orientierung R' der Astigmatismusachse zu verwenden.

**16.** Verfahren nach einem der Ansprüche 13 bis 15, **dadurch gekennzeichnet, dass** die aktuelle Orientierung (R') der Astigmatismusachse auf einem Display dargestellt wird und/oder in den Strahlengang eines Operationsmikroskops eingespiegelt wird.

## Claims

**1.** Device designed to display the astigmatism axis of the eye, comprising

an observation unit (10) for observing the eye (13),
a display unit (11) designed to display the orientation (R) of the astigmatism axis (A) of the eye (13) in an image (12) of the eye (13),
a sensor device (20) designed to generate sensor data that indicates a change in the orientation of the observation unit (10) relative to the eye (13), and
a computing unit (30),
**characterized in that**
the sensor device (20) comprises at least one rotation rate sensor (20a) which is attached to the observation unit (10) and which is designed to detect an angle of rotation of the observation unit (10) about its observation direction (O), and
the computing unit (30) is designed to use the sensor data of the rotation rate sensor (20a) to calculate from the orientation (R) of the astigmatism axis (A) in the image (12) of the eye (13) at a first point in time (T1) and from the rotation angle of the observation unit (10) around its observation direction (O) at the first point in time (T1) and at a current second point in time (T2), the current orientation (R') of the astigmatism axis (A) in the image (12) of the eye (13), and to provide it to the display unit (30) for display in the current image (12) of the eye (13).

**2.** Device according to claim 1, **characterized in that** the observation unit (10) comprises a surgical microscope or is configured as such.

**3.** Device according to claim 1 or 2, **characterized in that** the observation unit (10) comprises a device (10a) for capturing images of the eye (13) or is configured as such.

**4.** Device according to any one of the preceding claims, **characterized in that** the display unit (11) is configured as a display.

**5.** Device according to any one of the preceding claims, **characterized in that** the display unit (11) comprises a mirror arrangement for coupling into the optical path of the observation unit (10).

**6.** Device according to any one of the preceding claims, **characterized by** a memory (31) for storing a reference value representing the orientation (R) of the astigmatism axis (A) of the eye (13) at the first point in time (T1), and/or for storing a reference orientation of the observation unit (10).

**7.** Device according to any one of the preceding claims, **characterized in that** the sensor device (20) determines the position of the observation unit (10).

**8.** Device according to any one of the preceding claims, **characterized in that** the sensor device (20) comprises one or more inertial measurement units.

**9.** Device according to any one of the preceding claims, **characterized in that** the sensor device (20) comprises one or more sensors selected from the group consisting of acceleration sensors, direction sensors, compass sensors, and sensors (21) for measuring the angular position of the joints (10b) of the observation unit (10).

**10.** Device according to any one of the preceding claims, **characterized in that** the sensor device (20) comprises one or more sensors (22) for determining the orientation and/or position of the head (60) of the patient.

11. Device according to any one of the preceding claims, **characterized in that** the sensor device comprises one or more sensors (23) for determining the orientation and/or position of an operating table (40).

12. Device according to any one of the preceding claims, **characterized by** a device for tracking the position and/or orientation of the observation unit (10) and/or the orientation and/or position of the head (60) of the patient.

13. Method for displaying the astigmatism axis of the eye, **characterized by** the steps of:

> providing an observation unit (10) for observing the eye (13);
> providing the orientation (R) of the astigmatism axis of the eye (13) in an image (12) of the eye (13) for display on a display unit (11) during observation of the eye (13);
> acquiring and storing sensor data indicating a change in the orientation of the observation unit (10) relative to the eye (13);
> **characterized in that**
> the rotation angle of the observation unit (10) about its observation direction (O) is detected by a rotation rate sensor (20a), and
> using the sensor data of the rotation rate sensor (20a), the current orientation (R') of the astigmatism axis (A) in the image (12) of the eye (13) is calculated from the orientation (R) of the astigmatism axis (A) in the image (12) of the eye (13) at a first point in time (T1) and from the rotation angle of the observation unit (10) about its observation direction (O) at the first point in time (T1) and at a current second point in time (T2), and is provided to the display unit (30) for display in the current image (12) of the eye (13).

14. Method according to claim 13, **characterized in that** the orientation of the observation unit (10) is tracked.

15. Method according to claim 13 or 14, **characterized in that** the orientation and/or position of the head (60) of a patient and/or of a patient table (40) is measured in order to detect a rotation of the head (60) about the observation direction (O) or the optical axis of the observation unit (10) and to use it in calculating the current orientation (R') of the astigmatism axis.

16. Method according to any one of claims 13 to 15, **characterized in that** the current orientation (R') of the astigmatism axis is displayed on a display and/or is coupled into the optical path of a surgical microscope.

**Revendications**

1. Dispositif configuré pour afficher l'axe d'astigmatisme de l'œil, comprenant

> une unité d'observation (10) destinée à l'observation de l'œil (13);
> une unité d'affichage (11) configurée pour afficher l'orientation (R) de l'axe d'astigmatisme (A) de l'œil (13) dans une image (12) de l'œil (13);
> un dispositif de capteurs (20) configuré pour générer des données de capteurs indiquant une modification de l'orientation de l'unité d'observation (10) par rapport à l'œil (13); et
> une unité de calcul (30),
> **caractérisé en ce que**
> le dispositif de capteurs (20) comprend au moins un capteur de vitesse de rotation (20a), fixé à l'unité d'observation (10) et configuré pour détecter un angle de rotation de l'unité d'observation (10) autour de sa direction d'observation (O), et
> l'unité de calcul (30) est configurée pour calculer, à l'aide des données du capteur de vitesse de rotation (20a), à partir de l'orientation (R) de l'axe d'astigmatisme (A) dans l'image (12) de l'œil (13) à un premier instant (T1) et de l'angle de rotation de l'unité d'observation (10) autour de sa direction d'observation (O) au premier instant (T1) et à un second instant actuel (T2), l'orientation actuelle (R') de l'axe d'astigmatisme (A) dans l'image (12) de l'œil (13), et pour la fournir à l'unité d'affichage (30) en vue de son affichage dans l'image actuelle (12) de l'œil (13).

2. Dispositif selon la revendication 1, **caractérisé en ce que** l'unité d'observation (10) comprend un microscope chirurgical ou est configurée comme tel.

3. Dispositif selon la revendication 1 ou 2, **caractérisé en ce que** l'unité d'observation (10) comprend un dispositif (10a) pour la capture d'images de l'œil (13) ou est configurée comme tel.

**4.** Dispositif selon l'une quelconque des revendications précédentes, **caractérisé en ce que** l'unité d'affichage (11) est configurée comme un écran.

**5.** Dispositif selon l'une quelconque des revendications précédentes, **caractérisé en ce que** l'unité d'affichage (11) comprend un agencement de miroirs destiné à l'insertion dans le trajet optique de l'unité d'observation (10).

**6.** Dispositif selon l'une quelconque des revendications précédentes, **caractérisé par** une mémoire (31) destinée à mémoriser une valeur de référence représentant l'orientation (R) de l'axe d'astigmatisme (A) de l'œil (13) au premier instant (T1), et/ou à mémoriser une orientation de référence de l'unité d'observation (10).

**7.** Dispositif selon l'une quelconque des revendications précédentes, **caractérisé en ce que** le dispositif de capteurs (20) détermine la position de l'unité d'observation (10).

**8.** Dispositif selon l'une quelconque des revendications précédentes, **caractérisé en ce que** le dispositif de capteurs (20) comprend une ou plusieurs unités de mesure inertielle.

**9.** Dispositif selon l'une quelconque des revendications précédentes, **caractérisé en ce que** le dispositif de capteurs (20) comprend un ou plusieurs capteurs choisis dans le groupe constitué de capteurs d'accélération, capteurs de direction, capteurs de boussole et capteurs (21) destinés à mesurer la position angulaire des articulations (10b) de l'unité d'observation (10).

**10.** Dispositif selon l'une quelconque des revendications précédentes, **caractérisé en ce que** le dispositif de capteurs (20) comprend un ou plusieurs capteurs (22) destinés à déterminer l'orientation et/ou la position de la tête (60) du patient.

**11.** Dispositif selon l'une quelconque des revendications précédentes, **caractérisé en ce que** le dispositif de capteurs comprend un ou plusieurs capteurs (23) destinés à déterminer l'orientation et/ou la position d'une table d'opération (40).

**12.** Dispositif selon l'une quelconque des revendications précédentes, **caractérisé par** un dispositif de suivi de la position et/ou de l'orientation de l'unité d'observation (10) et/ou de l'orientation et/ou de la position de la tête (60) du patient.

**13.** Procédé pour afficher l'axe d'astigmatisme de l'œil, **caractérisé par** les étapes suivantes:

- fournir une unité d'observation (10) pour observer l'œil (13);
- fournir l'orientation (R) de l'axe d'astigmatisme de l'œil (13) dans une image (12) de l'œil (13) pour affichage sur une unité d'affichage (11) pendant l'observation de l'œil (13);
- acquérir et mémoriser des données de capteurs indiquant une modification de l'orientation de l'unité d'observation (10) par rapport à l'œil (13);

**caractérisé en ce que**
l'angle de rotation de l'unité d'observation (10) autour de sa direction d'observation (O) est détecté par un capteur de vitesse de rotation (20a), et à l'aide des données du capteur de vitesse de rotation (20a), l'orientation actuelle (R') de l'axe d'astigmatisme (A) dans l'image (12) de l'œil (13) est calculée à partir de l'orientation (R) de l'axe d'astigmatisme (A) à un premier instant (T1) et de l'angle de rotation de l'unité d'observation (10) autour de sa direction d'observation (O) au premier instant (T1) et à un second instant actuel (T2), et est fournie à l'unité d'affichage (30) pour affichage dans l'image actuelle (12) de l'œil (13).

**14.** Procédé selon la revendication 13, **caractérisé en ce que** l'orientation de l'unité d'observation (10) est suivie.

**15.** Procédé selon la revendication 13 ou 14, **caractérisé en ce que** l'orientation et/ou la position de la tête (60) d'un patient et/ou d'une table de patient (40) est mesurée afin de détecter une rotation de la tête (60) autour de la direction d'observation (O) ou de l'axe optique de l'unité d'observation (10) et d'en tenir compte lors du calcul de l'orientation actuelle (R') de l'axe d'astigmatisme.

**16.** Procédé selon l'une quelconque des revendications 13 à 15, **caractérisé en ce que** l'orientation actuelle (R') de l'axe d'astigmatisme est affichée sur un écran et/ou est insérée dans le trajet optique d'un microscope chirurgical.

Fig. 1

16

# Fig. 2

# Fig. 3A

Fig. 3B

Fig. 3C

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015176699 A2
- US 2011157553 A1
- WO 2012041349 A1
- US 20110019151 A1
- US 2011230751 A1
- US 2015077528 A1
- US 2008204864 A1

**In der Beschreibung aufgeführte Nicht-Patentliteratur**

- **BRUNA V. VENTURA et al.** Surgical management of astigmatism with toric intraocularlenses. *ARQUIVOS BRASILEIROS DE OFTALMOLOGIA*, vol. 77 (2), 125-131